(19) Europäisches Patentamt
European Patent Office
Office européen des brevets

(11) Numéro de publication : **0 291 390 B1**

# (12) FASCICULE DE BREVET EUROPEEN

(45) Date de publication du fascicule du brevet : **22.01.92 Bulletin 92/04**

(51) Int. Cl.$^5$ : **G01N 33/546,** C08F 291/00, C08F 257/02

(21) Numéro de dépôt : **88401079.4**

(22) Date de dépôt : **04.05.88**

## (54) Procédé de préparation de particules de polymère ou de latex constitués de particules de polymère comportant, implantées à leur surface, des molécules amphiphiles portant des groupes ionogènes ou réactifs.

(30) Priorité : **11.05.87 FR 8706550**

(43) Date de publication de la demande : **17.11.88 Bulletin 88/46**

(45) Mention de la délivrance du brevet : **22.01.92 Bulletin 92/04**

(84) Etats contractants désignés : **AT BE CH DE ES FR GB GR IT LI LU NL SE**

(56) Documents cités :
**EP-A- 0 055 132**
**EP-A- 0 169 434**
**DE-A- 2 529 937**
**FR-A- 1 537 741**
**FR-A- 2 450 263**

(73) Titulaire : **RHONE-POULENC CHIMIE**
**25, quai Paul Doumer**
**F-92408 Courbevoie Cédex (FR)**

(72) Inventeur : **Hogenmuller, Roger**
**3, Impasse de la Source**
**F-69110 Sainte Foy les Lyon (FR)**
Inventeur : **Chauvel, Bernard**
**24, Chemin de la Commanderie**
**F-95120 Ermont (FR)**
Inventeur : **Daniel, Jean-Claude**
**Résidence "Le Panorama" 13 rue de Neuilly**
**F-94120 Fontenay-sous-Bois (FR)**

(74) Mandataire : **Vignally, Noel et al**
**Rhône-Poulenc Chimie Service Brevets**
**Chimie Centre de Recherches de Saint-Fons**
**B.P. 62**
**F-69190 Saint-Fons Cédex (FR)**

EP 0 291 390 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de particules de polymère se présentant telles quelles ou en dispersion aqueuse, comportant, implantées à leur surface des molécules amphiphiles portant des groupes ionogènes ou réactifs. Les particules ou les latex de particules ainsi obtenus sont particulièrement intéressants en biologie.

Le but de la Demanderesse a été de préparer des particules de polymère portant des motifs ionogènes ou réactifs suffisamment éloignés de la surface desdites particules pour pouvoir fixer, sur lesdits motifs, des molécules encombrantes telles que les protéines et ce sans risque de modifier l'activité desdites molécules par contact avec la surface macromoléculaire.

Le brevet DE-A-2529937 décrit la préparation de réactifs latex pour des réactions sérologiques, consistant à adsorber un tensio-actif non-ionique, de type polymère d'oxyde d'éthylène, sur des particules de latex par mise en contact des dites particules de latex et du tensioactif en milieu aqueux, le mélange étant maintenu au repos et à température ambiante.

Ce procédé sommaire de simple adsorption ne semble pas de nature à fixer une quantité relativement importante de tensio-actif, ni à permettre une bonne cohésion de la liaison particules/tensio-actif.

Selon l'invention, ledit procédé est caractérisé par les étapes suivantes :

1) on fait diffuser dans une dispersion aqueuse "semence" de particules de polymère préalablement préparée, un amorceur organosoluble de polymérisation présentant une solubilité dans l'eau de l'ordre de $10^{-3}$ à $10^{-2}$ g/ litre d'eau, ladite étape étant réalisée à une température inférieure à celle de décomposition de l'amorceur ;
2) on fait gonfler les particules de la dispersion ainsi obtenue par introduction de la composition monomère à polymériser, ladite composition monomère à polymériser contenant au moins un monomère et un composé amphiphile de HLB supérieur ou égal à 10 et de masse moléculaire supérieure ou égale à 400, dont la séquence hydrophile est une séquence hydrophile oligomère terminée par au moins un groupe ionogène ou réactif, ladite étape étant réalisée avec une quantité de composition monomère inférieure à la quantité maximum de composition que peut absorber ladite semence, à une température inférieure à la température de décomposition de l'amorceur ;
3) on polymérise ladite composition monomère à une température au moins égale à celle de décomposition de l'amorceur ; la nature du polymère constituant la semence et celle du ou des monomères constituant la composition monomère étant telles que le polymère final présente une température de transition vitreuse Tg supérieure à environ 40°C, de préférence supérieure à 70°C
4) on élimine tout constituant amphiphile non implanté à la surface des particules en dispersion aqueuses ainsi obtenues
5) éventuellement on sépare les particules de polymère ainsi obtenues comportant, implantées à leur surface, des molécules dudit composé amphiphile.

Le ou les monomères constituant la composition monomère à polymériser et celui ou ceux dont dérivent les particules de la dispersion aqueuse "semence" peuvent être identiques ou différentes.

Ils peuvent être choisis parmi :

— les monomères vinylaromatiques (styrène, vinyltoluène)
— les alkylesters d'acides $\alpha$-$\beta$ insaturés (acrylates et méthacrylates de méthyle, éthyle)
— les esters d'acides carboxyliques insaturés (acétate de vinyle)
— le chlorure de vinyle ; le chlorure de vinylidène
— les diènes (butadiène)
— ceux présentant des fonctions nitriles (acrylonitrile)

La dispersion aqueuse "Semence" mise en oeuvre à la première étape présente un extrait sec de l'ordre de 1 à 40% en poids, de préférence de l'ordre de 10 à 25% en poids : elle est constituée de particules de polymère présentant un diamètre de l'ordre de 0,1 à 15 $\mu$m et généralement de l'ordre de 0,3 à 5 $\mu$m ; lesdites particules peuvent correspondre à une certaine distribution ou bien être calibrées.

Le polymère "semence" (extrait sec) peut représenter de 50 à 95% en poids, de préférence de 65 à 85% en poids du polymère final.

Une variante de réalisation dudit procédé consiste à mettre en oeuvre des dispersions aqueuses "semence" dont les particules sont magnétisables.

Lesdites particules magnétisables contiennent de 0,5 à 50% en poids (de préférence de 0,5 à 35% et tout particulièrement de 0,7 à 20%) d'une charge magnétique dont la taille est inférieure à 1 $\mu$m et de préférence comprise entre 0,002-0,05 $\mu$m ; la charge magnétique est bien évidemment suffisamment fine pour pouvoir être

incluse dans les particules de polymère.

Cette charge magnétique peut être constituée par exemple par :

- des métaux ou leurs alliages tels que : fer, fer-silicium, nickel, cobalt, ou leurs alliages avec du molybdène, du chrome, du cuivre, du vanadium, du manganèse, de l'aluminium, du titane ;
- des oxydes de fer : $Fe_3O_4$ ou $\gamma$-$Fe_2O_3$ pur ou en combinaison ou en mélange avec d'autres oxydes comme les oxydes de cobalt, manganèse, zinc, baryum, terres rares ;
- du dioxyde de chrome.

Lesdites dispersions aqueuses "semence" de particules magnétisables peuvent être obtenues selon les procédées décrits dans le brevet européen n° 38.730 et le brevet américain n° 4.157.323.

Il est particulièrement intéressant d'utiliser comme amorceur de polymérisation un amorceur liquide à la température de réalisation de la première étape, tel que le peroxyde de dioctanoyle, de didécanoyle, le peroxydicarbonate de dioctanoyle, de didecyle, de didodecyle.

La quantité d'amorceur mise en oeuvre correspond à environ 0,1 à 10%, de préférence de 1 à 5% en poids par rapport au poids de la composition monomère à polymériser.

L'étape de diffusion de l'amorceur dans la dispersion aqueuse "semence" peut être réalisée par mise en contact sous agitation de la dispersion aqueuse "semence" avec l'amorceur, ledit amorceur ayant été préémulsionné par homogénéisation dans de l'eau contenant un émulsifiant. Cette préémulsion peut être réalisée en mettant en oeuvre pour 100 g de particules sèches de "semence", de l'ordre de 200 à 2000 g d'une solution à 0,1-0,5% en poids d'un émulsifiant dans l'eau.

A titre d'exemples d'émulsifiant, on peut citer les alkylsulfates, alkylsufonates, alkylarylsulfonates alcalins.

Cette étape de diffusion est réalisée à une température inférieure à la température de décomposition de l'amorceur, mais suffisante pour que celui-ci soit à l'état liquide. Cette opération est généralement réalisée à une température de l'ordre de 25 à 45°C pendant environ 15 à 25 heures.

Les composés amphiphiles mis en oeuvre à la deuxième étape présentent de préférence un HLB supérieur ou égal à 10 et inférieur à 20. Ils présentent une séquence hydrophile oligomère comme par exemple une séquence polyoxyalkylénée contenant de 5 à 100, de préférence de 5 à 50, motifs oxyalkylénées en $C_2$-$C_3$, une séquence polycarboxyethylénée et/ou polyamidoéthylénée et/ou polycyanoéthylénée contenant de 4 à 50 motifs carboxyethylénés et/ou amidoéthylénés et/ou cyanoéthylénés, ladite séquence hydrophile oligomère étant terminée par un groupe ionogène ou réactif.

Parmi les terminaisons ionogènes ou réactives, ou peut citer les groupements : —OH, —$SO_3H$, —COOH, —CHO, —Ø$CH_2Cl$, —$NH_2$, —$NR_2$, —$NR_3$ (R étant un radical alkyle en $C_1$-$C_2$), —$CONH_2$, —NH-$NH_2$, —NH-NH-CO-$NH_2$, —SH,

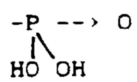

A titre d'exemple de composé amphiphile on peut citer :

— les alcools gras ou les acides gras polyoxyéthylénés et/ou polyoxypropylénés présentant de 5 à 50 motifs oxyalkylènes et dont la séquence hydrophobe contient environ de 8 à 20 atomes de carbone (tels que les CEMULSOL DB commercialisés par la Société Française d'Organo Synthèse, les SOPROPHOR LA commercialisés par Rhône-Poulenc)

— les esters desdits alcools gras portant des fonctions semicarbazides (tels que les esters desdits alcools et de l'acide phenylcarbamique meta-semicarbazide para-méthyle), des fonctions acides (tels que les monoesters desdits alcools et de l'acide azélaïque), des fonctions thiols (tels que les esters desdits alcools avec l'acide thioglycolique)

— les amides desdits acides gras (tels que les ETHOMID commercialisés per Armour Industrial Chemical Co)

— les alkylpyhénols polyoxyalkylénés et/ou polyoxypropylénés présentant de 5 à 50 motifs oxyalkylènes et dont le ou les radicaux alkyles contiennent de 8 à 12 atomes de carbone, et leurs esters avec l'acide phosphorique (tels que les SOPROPHOR BC et OP commercialisés par Rhône-Poulenc, les SOPROPHOR NFP commercialisés par Geronazzo, les GAFAC RE commercialisés par General Aniline and Film Corp.)

— les amines grasses polyoxyéthylénées dont la séquence hydrophobe contient de 8 à 22 atomes de carbone (telles que les SOPROMINE commercialisés par Rhône-Poulenc, les ETHOMEEN commercialisés par Armour Industrial Co)

La quantité de composé amphiphile mis en oeuvre est telle qu'elle représente environ de $10^{-5}$ à $10^{-1}$ mole,

de préférence de $10^{-4}$ à $10^{-2}$ mole pour 100 g de polymère final.

Le ou les monomère ainsi que le composé amphiphile sont introduits dans le milieu réactionnel issu de la première étape sous agitation.

L'étape de gonflement dure généralement de l'ordre de 1/2 à 4 heures ; elle est réalisée à une température du même ordre que celle de l'étape de diffusion.

La troisième étape, c'est-à-dire celle de polymérisation, est réalisée par élévation de température jusqu'à une température supérieure à celle de décomposition de l'amorceur.Cette opération de polymérisation dure généralement environ de 3 à 6 heures.

Si un composé organique insoluble autre que l'amorceur a été mis en oeuvre à la première étape, celui-ci peut être éliminé par exemple par évaporation sous vide.

Le milieu obtenu est alors une dispersion aqueuse de particules constituées de chaines macromoléculaires emmêlées dans leur couche périphérique avec la partie hydrophobe du composé amphiphile et fixant ainsi ledit composé amphiphile à la surface desdites particules.

Le composé amphiphile non implanté durant l'opération de polymérisation ainsi que tout constituant émulsifiant préalablement présent dans la dispersion sont ensuite éliminés par lavage aqueux, par exemple par ultrafiltration.

Les particules de polymère comportant le composé amphiphile implanté en surface ainsi obtenues peuvent être séparées si désiré du milieu aqueux par les méthodes classiques de sédimentation par centrifugation.

Les particules ou les latex de particules obtenus selon le procédé de l'invention sont particulièrement intéressants en biologie, pour la fixation de molécules biologiques (anticorps, antigènes) par covalence.

La fixation des molécules biologiques par covalence sur les particules de polymères peut être réalisée par réaction de couplage, réaction faisant intervenir les groupements terminaux de la molécule amphiphile implantée et les groupements fonctionnels de la molécule biologique à fixer.

Cette réaction de couplage peut être réalisée selon des méthodes bien connues, par exemple :

- en faisant appel à des agents de couplage (tels que glutaraldéhyde, carbodiimide hydrosoluble)
- par activation des fonctions du polymère (par exemple par diazotation, par action du bromure de cyanogène, de l'hydrazine) puis réaction avec la molécule à fixer.

Les produits peuvent donc être utilisés pour la réalisation de tests de diagnostic du type agglutination, radioimmunologique, enzymatique.

EXEMPLE 1

Implantation de CEMULSOL DB 25/18 (en abréviation CA-OH : composé amphiphile à fonction alcool), comme agent amphiphile, sur des particules de polystyrène.

Formule moyenne du composé amphiphile :

$$C_{18}H_{37}(O\text{-}CH_2\text{-}CH_2)_{50}OH$$

Dans un récipient de 500 ml, on charge 316,4 g d'eau, 1,54 g de peroxyde de dioctanoyle et 1,12 g de laurylsulfate de sodium. On chauffe à 30°C jusqu'à fusion du peroxyde de dioctanoyle. Le mélange est émulsionné à l'ultraturax pendant 1 minute à 20 000 t/mn en maintenant la température entre 25 et 35°C.

56 g de particules d'un latex "semence" de polystyrène, présentant un taux d'extrait sec de 30% en poids et constitué de particules calibrées de 0,8 μm sont ajoutés. L'ensemble est agité pendant 20 heures à 30°C.

20,44 g de styrène et 7,64 g de composé amphiphile (ce qui correspond à 4,09 mmole de composé amphiphile) sont ajoutés au mélange préparé. L'ensemble est agité pendant 2 heures à 40°C à 400 t/mn.

On augmente la température jusqu'à 70°C ; cette température est maintenue pendant 4 heures en agitant à 70 t/mn.

Le styrène non réagi est éliminé par stripping à 70°c sous une pression de 160 mm Hg dans un évaporateur rotatif.

Après refroidissement le latex obtenu est lavé par ultrafiltration à l'aide de 2,5 l d'eau épurée pour 10 g de particules de latex, pour éliminer les molécules de composé amphiphile non implanté ainsi que les émulsifiants présents dans le latex de départ.

Le latex reste stable après ultrafiltration, ce qui est bien un indice de l'implantation du composé amphiphile.

Le latex obtenu est dosé par résonnance magnétique nucléaire.

On constate que la quantité en poids d'agent amphiphile implanté dans 100 g de particules finales est de 7,8 (théorie = 9,1).

EXEMPLE 2

Implantation de l'ester de CEMULSOL DB 25/18 et de l'acide phénylcarbamique méta-semicarbazide para-méthyle, (en abréviation CASC : composé amphiphile à fonction semi-carbazide) comme agent amphiphile, sur les particules d'un latex calibré de polystyrène.

Formule moyenne du composé amphiphile :

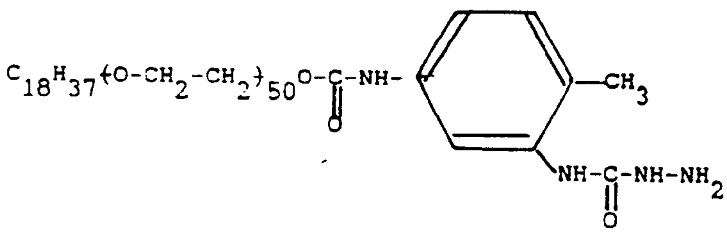

Préparation du composé amphiphile

Le composé est préparé en deux étapes :

— une première étape consistant à fonctionnaliser le CEMULSOL DB 25/18 par du toluène diisocyanate en présence de dioxanne comme solvant, à une température de 90°C

— une deuxième étape consistant à fonctionnaliser le CEMULSOL DB 25/18 ainsi obtenu par addition d'hydrate d'hydrazine en présence de dioxanne, à température ordinaire.

Le composé amphiphile recherché est précipité à froid dans de l'éther éthylique.

Implantation

On répète l'opération décrite à l'exemple 1 en remplaçant le CEMULSOL DB 25/18 par 7,64 g de son dérivé préparé comme ci-dessus indiqué, ce qui correspond à 3,78 mmole de composé amphiphile dispersé dans le styrène.

Le résultat du dosage par RMN figure au tableau I.

EXEMPLE 3

Implantation de l'ester de CEMULSOL DB 25/18 et de l'acide thioglycolique (en abréviation CA-SH : composé amphiphile à fonction thiol) comme agent amphiphile, sur les particules d'un latex calibré de polystyrène.

Formule moyenne du composé amphiphile

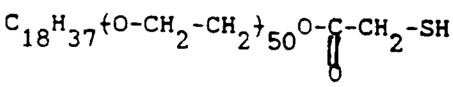

Ce composé peut être préparé selon le procédé décrit dansle brevet américain n° 2.461.920.

On répète l'opération décrite à l'exemple 1 en remplaçant le CEMULSOL DB 25/18 par 7,64 g de son ester thioglycolique de formule ci-dessus, ce qui correspond à 3,94 mmole de composé amphiphile.

Le résultat du dosage par conductrimétrie figure au tableau I.

EXEMPLE 4

Implantation du monoester de CEMULSOL DB 25/18 et de l'acide azélaïque, (en abréviation CA-COOH : composé amphiphile à fonction acide), comme composé amphiphile, sur les particules d'un latex calibré de polystyrène.

Formule moyenne du composé amphiphile :

$$C_{18}H_{37}\text{-}(O\text{-}CH_2\text{-}CH_2)_{50}\text{-}O\text{-}\underset{\underset{O}{\|}}{C}\text{-}(CH_2)_7\text{-}COOH$$

Celui-ci peut être préparé par estérification du CEMULSOL DB 25/18 par l'acide azélaïque dans la pyridine en présence de dicyclohexylcarbodiimide comme agent de condensation.

On répète l'opération décrite à l'exemple 1 en remplaçant le CEMULSOL DB 25/18 par 3,82 g de son ester de formule ci-dessus, ce qui correspond à 1,89 mmole de composé amphiphile.

Le résultat du dosage par RMN figure au tableau I.

## EXEMPLE 5

Implantation de CEMULSOL DB 25/18 comme agent amphiphile sur des particules calibrées de copolymère styrène/méthacrylate de méthyle 40/60 en poids.

On répète l'opération décrite à l'exemple 1 en remplaçant poids pour poids le latex "semence" de polystyrène (56 g de particules) par un latex "semence" de copolymère styrène/méthacrylate de méthyle 40/60 en poids (56 g de particules) présentant un taux d'extrait sec de 33% et constitué de particules calibrées de 0,705 µm.

La nature et les quantités des autres réactifs mis en oeuvre sont les mêmes que ceux de l'exemple 1 (c'est-à-dire 20,44 g de styrène comme composition monomère) ; il en est de même des conditions opératoires.

Le résultat du dosage par RMN est donné au tableau I.

## Exemple 6

Les particules de latex obtenues à l'exemple 1 sont sédimentées à l'aide d'une centrifugeuse BECKMAN L 50 munie d'un rotor 30 (appareil commercialisé per BECKMAN) tournant à 10.000 t/mn pendant 15 mn, puis séchées à l'étuve sous vide à 40°C et conservées sous azote.

Les caractéristiques des particules sont conservées.

Les abréviations relatives au polymère de départ figurant dans le tableau ont la signification suivante :

PS : polystyrène

P(S/MAM) : copolymère styrène/méthacrylate de méthyle

ES : extrait sec

S signifie styrène.

TABLEAU 1

| EXEMPLE | | 1 | 2 | 3 | 4 | 5 |
|---|---|---|---|---|---|---|
| LATEX | polymère | PS | PS | PS | PS | P(S/MAM) |
| | Ø en µm | 0,8 | 0,8 | 0,8 | 0,8 | 0,705 |
| | ES % | 30 | 30 | 30 | 30 | 33 |
| monomère | | S | S | S | S | S |
| COMPOSE AMPHIPHILE | nature | CA-OH | CASC | CA-SH | CA-COOH | CA-OH |
| | g/100 g de polymère + monomère | 10 | 10 | 10 | 5 | 10 |
| | mole/100 g de polymère + monomère | 5,35 | 4,95 | 5,16 | 2,47 | 5,35 |
| % implanté | . théorie | 9,1 | 9,1 | 9,1 | 4,8 | 9,1 |
| | . dosé | 7,8 | 7,9 | 5,2 | 3,3 | 7,2 |

## Revendications

1. Procédé de préparation de particules de polymère comportant, implantées à leur surface des molécules amphiphiles portant des groupes ionogènes ou réactifs, caractérisé par les étapes suivantes :

a) on fait diffuser dans une dispersion aqueuse "semence" de particules de polymère préalablement préparée, un amorceur organosoluble de polymérisation présentant une solubilité dans l'eau de l'ordre de $10^{-3}$ à $10^{-2}$ g/ litre d'eau, ladite étape étant réalisée à une température inférieure à celle de décomposition de l'amorceur ;

b) on fait gonfler les particules de la dispersion ainsi obtenue par introduction de la composition monomère à polymériser, ladite composition monomère à polymériser contenant au moins un monomère et un composé amphiphile de HLB supérieur ou égal à 10 et de masse moléculaire supérieure ou égale à 400, dont la séquence hydrophile est une séquence hydrophile oligomère terminée par au moins un groupe ionogène ou réactif, ladite étape étant réalisée avec une quantité de composition monomère inférieure à la quantité maximum de composition que peut absorber ladite semence, à une température inférieure à la température de décomposition de l'amorceur ;

c) on polymérise ladite composition monomère à une température au moins égale à celle de décomposition de l'amorceur ; la nature du polymère constituant la semence et celle du ou des monomères constituant

la composition monomère étant telles que le polymère final présente une température de transition vitreuse Tg supérieure à environ 40°C

d) on élimine tout constituant amphiphile non implanté à la surface des particules en dispersion aqueuses ainsi obtenues

e) éventuellement on sépare les particules de polymère ainsi obtenues comportant, implantées à leur surface, des molécules dudit composé amphiphile.

2. Procédé selon la revendication 1 caractérisé en ce que le polymère "semence" contient des motifs dérivés des monomères vinylaromatiques, des alkylesters d'acides $\alpha$-$\beta$ insaturés, des esters d'acides carboxyliques insaturés, du chlorure de vinyle, du chlorure de vinylidéne, des diènes, de ceux présentant des fonctions nitriles.

3. Procédé selon la revendication 1 ou 2 caractérisé en ce que le ou les monomeres à polymériser sont les monomères vinylaromatiques, les alkylesters d'acides $\alpha$-$\beta$ insaturés, les esters d'acides carboxyliques insaturés, le chlorure de vinyle, le chlorure de vinylidène, les diènes, ceux présentant des fonctions nitriles.

4. Procédé selon l'une quelconque des revendications précédentes caractérisé en ce que la dispersion aqueuse "semence" présente un extrait sec de l'ordre de 1 à 40% en poids et est constituée de particules de diamètre de l'ordre de 0,1 à 15 $\mu$m.

5. Procédé selon l'une quelconque des revendications précédentes caractérisé en ce que le polymère "semence" représente de 50 à 95 % en poids du polymère final.

6. Procédé selon l'une quelconque des revendications précédentes caractérisé en ce que les particules de la dispersion aqueuse "semence" sont magnétisables.

7. Procédé selon l'une quelconque des revendications précédentes caractérisé en ce que l'amorceur est du peroxyde de dioctanoyle, de didécanoyle, le peroxydicarbonate de dioctanoyle, de didécyle, de didodécyle.

8. Procédé selon l'une quelconque des revendications précédentes caractérisé en ce que la quantité d'amorceur correspond à environ 0,1 à 10% en poids par rapport au poids de la composition monomère à polymériser.

9. Procédé selon l'une quelconque des revendications précédentes caractérisé en ce que l'étape de diffusion de l'amorceur dans la dispersion aqueuse "semence" est réalisée par mise en contact sous agitation de la dispersion aqueuse "semence" avec l'amorceur, ledit amorceur ayant été préemulsionné par homogénéisation dans de l'eau contenant un émulsifiant.

10. Procédé selon l'une quelconque des revendications précédentes caractérisé en ce que l'étape de diffusion est réalisée à une température inférieure à la température de décomposition de l'amorceur, mais suffisante pour que celui-ci soit à l'état liquide.

11. Procédé selon l'une quelconque des revendications précédentes caractérisé en ce que le composé amphiphile présente une séquence hydrophile oligomère polyoxyalkylénée contenant de 5 à 100 motifs oxyalkylénés en $C_2$-$C_3$, une séquence polycarboxyethylénée et/ou polyamidoéthylénée et/ou polycyanoéthylénée contenant de 4 à 50 motifs carboxyéthylénés et/ou amidoéthylénées et/ou cyanoéthylénés, ladite séquence hydrophile oligomère étant terminée par un groupe —OH, —$SO_3H$, —COOH, —CHO, —Ø$CH_2Cl$, —$NH_2$, —$NR_2$, $NR_3$ (R étant un radical alkyle en $C_1$-$C_2$), —$CONH_2$, —NH-$NH_2$, —NH-NH-CO-$NH_2$, —SH,

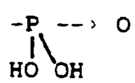

12. Procédé selon l'une quelconque des revendications précédentes caractérisé en ce que la quantité de composé de composé amphiphile est telle qu'elle représente environ de $10^{-5}$ à $10^{-1}$ mole pour 100 g de polymère final.

13. Procédé selon l'une quelconque des revendications précédentes caractérisé en ce que l'étape de gonflement est réalisée à une température du même ordre que celle de l'étape de diffusion.

14. Procédé selon l'une quelconque des revendications précédentes caractérisé en ce que l'étape de polymérisation est réalisée à une température supérieure à celle de décomposition de l'amorceur.

15. Utilisation des particules ou des dispersions aqueuses de particules obtenues selon le procédé faisant l'objet de l'une quelconque des revendications 1 à 14, en biologie.

## Claims

1. Process for the preparation of polymer particles comprising, implanted at their surface, amphiphilic molecules bearing ion-forming or reactive groups, characterised by the following stages :

a) an organosoluble polymerisation initiator which has a solubility in water of the order of $10^{-3}$ to $10^{-2}$ g/litre of water is caused to diffuse into a previously prepared aqueous "seed" dispersion of polymer particles, the said stage being carried out at a temperature below that of decomposition of the initiator ;

b) the particles of the dispersion thus obtained are swollen by introducing the monomeric composition to be polymerised, the said monomeric composition to be polymerised containing at least one monomer and an amphiphilic compound of HLB higher than or equal to 10 and with a molecular mass higher than or equal to 400, in which the hydrophilic block is an oligomeric hydrophilic block ending in at least one ion-forming or reactive group, the said stage being carried out with a quantity of monomeric composition smaller than the maximum quantity of composition which the said seed can absorb, at a temperature below the temperature of decomposition of the initiator ;

c) the said monomeric composition is polymerised at a temperature which is at least equal to that of decomposition of the initiator ; the nature of the polymer forming the seed and that of the monomer(s) forming the monomeric composition being such that the final polymer has a glass transition temperature Tg higher than approximately 40°C

d) any amphiphilic constituent which is not implanted at the surface of the particles which are thus obtained in aqueous dispersion is removed

e) the polymer particles thus obtained comprising molecules of the said amphiphilic compound implanted at their surface are optionally isolated.

2. Process according to Claim 1, characterised in that the "seed" polymer contains units derived from vinylaromatic monomers, alkyl esters of α,β-unsaturated acids, unsaturated carboxylic acid esters, vinyl chloride, vinylidene chloride, dienes or from those containing nitrile functional groups.

3. Process according to Claim 1 or 2, characterisedin that the monomer(s) to be polymerised are vinylaromatic monomers, alkyl esters of α,β-unsaturated acids, unsaturated carboxylic acid esters, vinyl chloride, vinylidene chloride, dienes, or those containing nitrile functional groups.

4. Process according to any one of the preceding claims, characterised in that the aqueous "seed" dispersion has a solids content of the order of 1 to 40% by weight and consists of particles of diameter of the order of 0.1 to 15 μm.

5. Process according to any one of the preceding claims, characterised in that the "seed" polymer represents from 50 to 95% by weight of the final polymer.

6. Process according to any one of the preceding claims, characterised in that the particles of the aqueous "seed" dispersion are magnetisable.

7. Process according to any one of the preceding claims, characterised in that the initiator is dioctanoyl or didecanoyl peroxide or dioctanoyl, didecyl or didodecyl peroxydicarbonate.

8. Process according to any one of the preceding claims, characterised in that the quantity of initiator corresponds to approximately 0.1 to 10% by weight relative to the weight of the monomeric composition to be polymerised.

9. Process according to any one of the preceding claims, characterised in that the stage of diffusion of the initiator in the aqueous "seed" dispersion is carried out by bringing the aqueous "seed" dispersion into contact with the initiator with stirring, the said initiator having been premulsified by homogenisation in water containing an emulsifier.

10. Process according to any one of the preceding claims, characterised in that the diffusion stage is carried out at a temperature below the temperature of decomposition of the initiator but sufficient for the latter to be in the liquid state.

11. Process according to any one of the preceding claims, characterised in that the amphiphilic compound contains a polyoxyalkylene oligomeric hydrophilic block containing from 5 to 100 $C_2$-$C_3$ oxyalkylene unite, a polycarboxyethylene and/or polyamidoethylene and/or polycyanoethylene block containing from 4 to 50 carboxyethylene and/or amidoethylene and/or cyanoethylene units, the said oligomeric hydrophilic block ending in an —OH, —$SO_3H$, —COOH, —CHO, —Ø$CH_2Cl$, —$NH_2$, —$NR_2$, $NR_3$ (R being a $C_1$-$C_2$ alkyl radical), —$CONH_2$, —NH-$NH_2$, —NH-NH-CO-$NH_2$, —SH

or 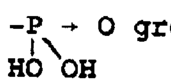 group.

12. Process according to any one of the preceding claims, characterised in that the quantity of amphiphilic compound is such that it represents approximately from $10^{-5}$ to $10^{-1}$ mol per 100 g of final polymer.

13. Process according to any one of the preceding claims, characterised in that the swelling stage is carried out at a temperature of the same order as that of the diffusion stage.

14. Process according to any one of the preceding claims, characterised in that the polymerisation stage is carried out at a temperature above that of decomposition of the initiator.

15. Use of the particles or of the aqueous dispersions of particles obtained according to the process forming the subject of any one of Claims 1 to 14, in biology.

## Patentansprüche

1. Verfahren zur Herstellung von Polymerenteilchen, die, an ihrer Oberfläche implantiert, amphiphile Moleküle mit ionogenen oder reaktiven Gruppen enthalten, gekennzeichnet durch die folgenden Stufen :

(a) man läßt in eine zuvor hergestellte, wäßrige "Keim"-Dispersion aus Polymerenteilchen einen organisch löslichen Polymerisationsstarter diffundieren, der eine Löslichkeit in Wasser in der Größenordnung von $10^{-3}$ bis $10^{-2}$ g/l Wasser besitzt, wobei diese Stufe bei einer Temperatur unterhalb derjenigen der Zersetzung des Starters durchgeführt wird ;

(b) man läßt sich die Teilchen der so erhaltenen Dispersion durch Einbringen der zu polymérisierenden Monomerenzusammensetzung vergrößern, wobei diese zu polymerisierende Monomerenzusammensetzung zumindest ein Monomeres und eine amphiphile Verbindung mit einem HLB von höher als oder gleich 10 und einer molaren Masse von höher als oder gleich 400 enthält, deren hydrophile Sequenz eine oligomere, hydrophile, mit zumindest einer ionogenen oder reaktiven Gruppe endende Sequenz ist, wobei die Stufe mit einer Menge der Monomerenzusammensetzung, die geringer ist als die maximale Menge der Zusammensetzung, die diesen Keim absorbieren kann, bei einer Temperatur unterhalb der Zersetzungstemperatur des Starters durchgeführt wird ;

(c) man polymerisiert diese Monomerenzusammensetzung bei einer Temperatur, die zumindest gleich der Zersetzungstemperatur des Starters ist, wobei die Natur des den Keim bildenden Polymeren und diejenigedes oder der die Monomerenzusammensetzung bildenden Monomeren derart ist, daß das endgültige Polymere eine Glasübergangstemperatur Tg von höher als etwa 40°C besitzt ;

(d) man entfernt sämtlichen, nicht an der Oberfläche der so erhaltenen Teilchen in der wäßrigen Dispersion implantierten, amphiphilen Bestandteil ;

(e) man trennt gegebenenfalls die so erhaltenen Polymerenteilchen, die, an ihrer Oberfläche implantiert, Moleküle dieser amphiphilen Verbindung enthalten, ab.

2. Verfahren gemäß Anspruch 1, dadurch gekennzeichnet, daß das "Keim"-Polymere Gruppen enthält, die sich von vinylaromatischen Monomeren, von Alkylestern $\alpha,\beta$-ungesättigter Säuren, von ungesättigten Erstern von Carbonsäuren, von Vinylchlorid, von Vinylidenchlorid, von Dienen, von solchen, die Nitrilfunktionen enthalten, ableiten.

3. Verfahren gemäß Anspruch 1 oder 2, dadurch gekennzeichnet, daß das oder die zu polymerisierenden Monomeren vinylaromatische Monomere, Alkylester $\alpha,\beta$-ungesättigter Säuren, ungesättigte Ester von Carbonsäuren, Vinylchlorid, Vinylidenchlorid, Diene, solche mit Nitril-funktionen sind.

4. Verfahren gemäß einem der vorhergehenden Ansprüche, dadurch gekennzeichnet, daß die wäßrige "Keim"-Dispersion einen Trockenextraktgehalt in der Größenordnung von 1 bis 40 Gew.% aufweist und aus Teilchen mit einem Durchmesser in der Größenordnung von 0,1 bis 15 $\mu$m besteht.

5. Verfahren gemäß einem der vorhergehenden Ansprüche, dadurch gekennzeichnet, daß das "Keim"-Polymere 50 bis 95 Gew.% des endgültigen Polymeren ausmacht.

6. Verfahren gemäß einem der vorhergehenden Ansprüche, dadurch gekennzeichnet, daß die Teilchen der wäßrigen "Keim"-Dispersion magnetisierbar sind.

7. Verfahren gemäß einem der vorhergehenden Ansprüche, dadurch gekennzeichnet, daß der Starter Dioctanoyl- oder Didecanoylperoxid, Dioctanoyl-, Didecyl- oder Didodecylperoxydicarbonat ist.

8. Verfahren gemäß einem der vorhergehenen Ansprüche, dadurch gekennzeichnt, daß die Menge des Starters etwa 0,1 bis 10 Gew.%, bezogen auf das Gewicht der zu polymerisierenden Monomerenzusammensetzung, beträgt.

9. Verfahren gemäß einem der vorhergehenden Ansprüche, dadurch gekennzeichent, daß die Stufe der Diffusion des Starters in die wäßrige "Keim"-Dispersion durch In-Kontakt-Brigen unter Rühren der wäßrigen "Keim"-Dispersion mit dem Starter durchgeführt wird, wobei der Starter durch Homogenisierung in ein Emulgiermittel enthaltendem Wasser prä-emulgiert worden ist.

10. Verfahren gemäß einem der vorhergehenden Ansprüche, dadurch gekennzeichnet, daß die Stufe der Diffusion bei einer Temperatur unterhalb der Zersetzungstemperatur des Starters, jedoch ausreichenden Temperatur, damit dieser sich in flüssigem Zustand befindet, duchgeführt wird.

11. Verfahren gemäß einem der vorhergehenden Ansprüche, dadurch gekennzeichnet, daß die amphiphile Verbindung eine Oligomere hydrophile Polyoxyalkylen-Sequenz mit 5 bis 100 $C_{2-3}$-Oxyalkylengruppen, eine

Polycarboxyethylen- und/oder Polyamidoethylen- und/oder Polycyanoethylen-Sequenz mit 4 mit 50 Carboxyethylen- und/oder Amidoethylen- und/oder Cyanoethylengruppen aufweist, wobei die oligomere hydrophile Sequenz mit einer Gruppe —OH, —$SO_3H$, —COOH, —CHO, —Ø$CH_2Cl$, —$NH_2$, —$NR_2$, $NR_3$ (R bedeutet einen $C_{1-2}$-Alkylrest), —$CONH_2$, —NH-$NH_2$, —NH-NH-CO-$NH_2$,

-SH, 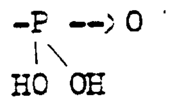

beendet ist.

12. Verfahren gemäß einem der vorhergehenden Ansprüche, dadurch gekennzeichnet, daß die Menge der amphiphilen Verbindung derart ist, daß sie etwa $10^{-5}$ bis $10^{-1}$ Mol je 100 g endgültiges Polymers ausmacht.

13. Verfahren gemäß der vorhergehenden Ansprüche, dadurch gekennzeichnet, daß die Stufe der Vergrößerung bei einer Temperatur der gleichen Größenordnung wie derjenigen bei der Diffusionsstufe durchgeführt wird.

14. Verfahren gemäß einem der vorhergehenden Ansprüche, dadurch gekennzeichnet, daß die Stufe der Polymerisation bei einer Temperatur durchgeführt wird, die höher als diejenige der Zersetzung des Starters.

15. Verwendung der gemäß einem der Ansprüche 1 bis 14 erhaltenen Teilchen oder wäßrigen Dispersionen der Teilchen in der Biologie.